## Europäisches Patentamt

## European Patent Office

## Office européen des brevets

(19)

(11) Veröffentlichungsnummer: **0 263 062 B1**

(12) **EUROPÄISCHE PATENTSCHRIFT**

(45) Veröffentlichungstag der Patentschrift: 01.08.90

(51) Int. Cl.⁵: **A61B 5/11**, G01N 33/15, G01B 11/28

(21) Anmeldenummer: 87810499.1

(22) Anmeldetag: 01.09.87

(54) Verfahren und Vorrichtung zur Aufzeichnung und/oder Auswertung des Bewegungsverhaltens von Versuchstieren.

(30) Priorität: 04.09.86 CH 3560/86

(43) Veröffentlichungstag der Anmeldung: 06.04.88 Patentblatt 88/14

(45) Bekanntmachung des Hinweises auf die Patenterteilung: 01.08.90 Patentblatt 90/31

(84) Benannte Vertragsstaaten: CH DE FR GB LI

(56) Entgegenhaltungen:
WO-A-86/04802
DE-A- 2 728 894
FR-A- 1 519 720
US-A- 3 378 675

MEDICAL AND BIOLOGICAL ENGINEERING, Band 11, Nr.4, Juli 1973, Seiten 490-498, Stevenhage, GB; A.COHEN et al.: "A small-animal activity-analysing system for behavioural studies"
NASA TECH.BRIEF, Nr. NTN-77/0819, Winter 1976, LAR-11999, US; D.L.GRAY et al: "Miniature angular position transducer"
JOURNAL OF PHARMACOLOGY AND EXPERIMENTAL THERAPEUTICS, Band 125, Nr.237, 1959, Seiten 237-240 L.C.HENDERSHOT et al.: "Atagonism of the frequency of phenylquinone-induced writhing in the

(73) Patentinhaber: CIBA-GEIGY AG, Klybeckstrasse 141, CH-4002 Basel(CH)

(72) Erfinder: Brom, Richard, 11, rue Bellevue, F-68440 Habsheim/Brübach(FR)
Erfinder: Schweizer, Alfred, Dr., Nufenenstrasse 20, CH-4054 Basel(CH)
Erfinder: Bichsel, Werner, Eichenweg 16, CH-4132 Muttenz(CH)

(56) Entgegenhaltungen: (Fortsetzung)
mouse by weak aanalgesics and non analgesics"
Optoelectronics: An Introduction, P. 310-312; J.WILSOPN, HAWKES Prentice Hall int.
PATENT ABSTRACTS OF JAPAN, Band 10, no. 74 (P-439)(2131), 25 März 1986 & JP-A-60213808 (HOKUSAN K.K.) 26-10-85
PATENT ABSTRACTS OF JAPAN, Band 7, no. 286 (P-244)(1431), 21 Dezember 1983 & JP-A-58161806 (MITSUBISHI DENKI K.K.) 26-09-1983

ACTORUM AG

## Beschreibung

Die Erfindung betrifft ein Verfahren gemäss dem Oberbegriff des Patentanspruchs 1 und eine Vorrichtung zur Durchführung dieses Verfahrens.

Das Bewegungs- und Streckverhalten von Versuchstieren ist für verschiedene Tests signifikant. Beispielsweise wird der Streck-Test (Writhing-Test) zur Erfassung von schmerzdämpfenden Wirkungen von Pharmaka verwendet. Zu diesem Zweck wird ein Reizstoff (Irritans) in Peritoneum von Versuchstieren, z.B. Mäusen, injiziert, welcher eine geringe Schmerzen verursachende Entzündung auslöst. Mäuse reagieren auf diesen Reiz mit Streckungen, indem sie die Wirbelsäule nach unten drücken, was vermutlich eine momentane Erleichterung verursacht. Die Wirkung eines Pharmakons kann nun dadurch getestet werden, dass man die Anzahl der Streckbewegungen von Tieren, welche mit dem Pharmakon (Testsubstanz) behandelt wurden, mit der Anzahl der Streckbewegungen von nicht behandelten Tieren vergleicht (Journal of Pharmacology and Experimental Therapeutics, Vol. 125, Nr. 237, 1959, Seiten 237-240, L.C. Hedershot et al.). Analog können verschiedene Testpräparate, und insbesondere auch zentral und peripher wirkende Pharmaka miteinander verglichen werden.

Bislang wurden Streck-Tests visuell überwacht. Die Anzahl der signifikanten Streckbewegungen wurde mit Hilfe einer Stoppuhr gezählt und in Protokolle eingetragen. Die Resultate hängen dabei sehr stark von der beobachtenden Person und deren Ermüdungsgrad ab. Nachträgliche Kontrollen sind nicht möglich. Aus diesem Grunde sind die durch beobachtende Personen ermittelten Ergebnisse nicht objektiv, denn individuelle menschliche Fehlleistungen führen zu Protokollen, welche nicht immer ausreichend genau und aussagekräftig sind. Die von Beobachtungspersonen erstellten Protokolle können wegen dieser Unwägbarkeiten auch häufig nicht nachträglich als Beleg für die vorschriftsgemässe Durchführung der Tests dienen, um nachzuweisen, dass diese auch den immer grösser werdenden Anforderungen amtlicher Stellen, wie z.B. den GLP (Good Laboratory Practice) Normen der USA FDA (Food & Drug Administration) genügen.

Um die beim Einsatz von Beobachtungspersonen auftretenden Nachteile zu beseitigen, sind in der DE-A1-2 728 894 bereits ein Verfahren und eine Vorrichtung zur Registrierung der Bewegungsaktivität von Tieren auf optisch-optoelektronischer Grundlage in der DE-A1-2 728 894 vorgeschlagen worden. Dabei wird mittels einer optischen Linse ein Bild des zu beobachtenden Tieres auf eine aus einer Vielzahl von Photowiderständen bestehende lichtempfindliche Messplatte projiziert. die Photowiderstände sind in vier Gruppen aufgeteilt und liegen jeweils in einem Zweig einer Wheatston'schen Messbrücke. Je nach der Lage der einem Zweig der Messbrücke bzw. einer Gruppe zugeordneten Photowiderstände kann eine Bewegung des Tieres in eine bestimmte Richtung erfasst werden, wenn die bei der Bewegung auftretende Verschiebung des Randes der Abbildung des Tieres so weit verschoben wird, dass wenigstens in einem Photowiderstand eine messbare Änderung des elektrischen Stromes der Messbrücke hervorgerufen wird. Nachteilig ist es dabei, dass die Auflösung vom Abstand und von der Zahl der verwendeten Photowiderstände abhängt, weshalb brauchbare Ergebnisse nur mit einem hohen Verdrahtungsaufwand und einer Vielzahl von Photowiderständen erreicht werden können. Infolge der matrixartigen Anordnung der Photowiderstände ergeben sich für Bewegungen mit Ortsveränderungen (Lokomotionen) Vorzugsrichtungen, d.h. Richtungen, in denen die Erfassung einer Bewegung zu einer grösseren Stromänderung führt als in einer anderen Richtung. Wenn Bewegungen am Ort (Lokalmotilität) zur automatisierten Durchführung von Streck-Tests erfasst werden sollen, stört eine derartige Richtungsempfindlichkeit und führt je nach der Richtung der Streckbewegungen der Tiere zu Messfehlern.

In Medical and Biological Engineering, Vol. 11, Nr. 4, Juli 1973, Seiten 490 bis 498, A. Cohen et al. ist ein Aktivitätsanalysesystem für Verhaltensstudien von Kleintieren beschrieben, bei dem drei Matrixanordnungen vorgesehen sind, die jeweils eine Vielzahl von Leuchtdioden und Photodetektoren enthalten. Die Photodetektoren einer Matrixanordnung sind dabei jeweils parallel geschaltet, wobei das Ausgangssignal einem Schwellenwertdetektor zugeführt wird. Infolge der verhältnismässig grossen Abstände zwischen den einzelnen Elementen der Matrixanordnungen ergibt sich ein sehr geringes Auflösungsvermögen, so dass der Einsatz des bekannten Aktivitätsanalysesystems, das über eine Schnittstelle mit einem Minicomputer verbunden sein kann, die Erfassung von Streckbewegungen von Tieren nicht gestattet.

Ein aus der FR-A-1 519 720 bekannter automatischer Analysator für das Tierverhalten verfügt neben einer Reihe von mechanischen und akustischen Sensoren über mehrere Lichtquellen, die an einer Seitenwand eines Käfigs angeordnet sind und denen auf der gegenüberliegenden Seite photoelektrische Sensoren zugeordnet sind, deren Widerstand- sich in Abhängigkeit von der Intensität des empfangenen Lichtes verändert. Das Weglaufen einer Ratte wird durch den Analysator dadurch erfasst, dass die verschiedenen Lichtstrahlbündel zwischen den photoelektrischen Elementen unterbrochen werden, wobei Veränderungen des durch die photoelektrischen Sensoren fliessenden Stroms in einem Verstärker verstärkt werden und einen Zähler beaufschlagen. Verhältnismässig langsame Streckbewegungen kleiner Amplitude eines Versuchstieres können nicht erfasst werden.

Eine weitere Vorrichtung zur automatischen Auswertung der von mit Medikamenten behandelten Tieren ist in der WO-A 8 604 802 beschrieben. Mit dieser Vorrichtung werden die einen vorgegebenen Schwellenwert überschreitenden Vertikalbewegungen eines Stabes erfasst, an dem das Versuchstier vorzugsweise mit seinem Schwanz angebunden ist. Eine Auswertung eines optischen Bildes erfolgt in dem angeschlossenen Mikrocomputer jedoch nicht.

In der US-A 3 378 675 ist eine Vorrichtung zur automatischen Erfassung des Bewegungsverhaltens von Versuchstieren beschrieben, bei der das Tier in einen zylindrischen Metallbehälter eingebracht wird. Die Vorrichtung weist eine oberhalb des Versuchstieres angeordnete Projektionsfläche auf, die mit einem Raster von kleinen Oeffnungen versehen ist. Hinter jeder dieser Oeffnungen ist eine Silizium-Photodiode angeordnet. Alle Photodioden sind parallel geschaltet und über eine Differentiationsschaltung mit einem elektro-mechanischen Zählwerk verbunden. Die Photodioden sind in Sperrrichtung vorgespannt. Je nach der Anzahl und der überstrichenen Fläche der Photodioden, die mit dem vom Versuchstier reflektierten Licht beaufschlagt sind, ändert sich der Gesamtwiderstand der parallel geschalteten Photodioden, die als eine Anzahl parallel geschalteter Schalter fungieren. Die daraus resultierende Aenderung des Stromflusses ist im wesentlichen proportional zu der vom Bild des Versuchstiers auf der Projektionsfläche eingenommenen Fläche. Die Differentiationsschaltung detektiert jede dieser Aenderungen und generiert jeweils einen Impuls, welcher schliesslich das elektromechanische Zählwerk betätigt. Die einzelnen diskreten Photodioden sind in einem gewissen Abstand voneinander angeordnet und erfassen demgemäss nicht das gesamte Bild des Versuchstiers. Insbesondere kleinere Bewegungen im "Totraum" der Photodioden, das ist im Bereich des Zwischenraumes zwischen zwei benachbarten Photodioden, werden vielfach nicht erfasst und damit das Messergebnis verfälscht. Die gewählte Anordnung der Photodioden in einem bestimmten Raster legt auch eine bestimmte Mindestgrösse beziehungsweise -länge des Versuchstieres fest. Dieses muss mindestens so gross sein, dass die Länge seines Abbildes auf der Projektionsfläche etwa dem Abstand zwischen zwei benachbarten Photodioden entspricht. Es ist zwar denkbar den Diodenabstand an die Grösse des Versuchstieres anzupassen, jedoch stösst man dabei zwangsläufig an die Grenze der Ausdehnung der einzelnen Photodioden. Insbesondere aber würde dabei der Aufwand für die Verkabelung der einzelnen Photodioden ein nicht mehr vertretbares Mass erreichen.

Der Erfindung liegt daher die Aufgabe zugrunde, ein Verfahren und eine Vorrichtung zu schaffen, die es gestatten, das Streck- und Bewegungsverhalten von Versuchstieren zur Aufzeichnung und anschliessenden Auswertung optoelektronisch mit einem kontinuierlichen Auflösungsvermögen und gleicher Empfindlichkeit in alle Richtungen zu erfassen, wobei der Verdrahtungsaufwand der optoelektronischen Bauelemente auch bei einem grossen Gesichtsfeld klein sein soll.

Die Lösung dieser Aufgabe erfolgt durch ein Verfahren gemäss Kennzeichen des Patentanspruchs 1 und durch erfindungsgemässe Vorrichtungen zur Durchführung des Verfahrens, welche die im Kennzeichen des Patentanspruchs 9 bzw. des Patentanspruchs angeführten Merkmale aufweisen.

Zweckmässige Ausgestaltungen und Weiterbildungen der Erfindung ergeben sich aus den Unteransprüchen.

Im folgenden wird die Erfindung anhand der Figuren beispielsweise näher erläutert; es zeigen:

Fig. 1a und 1b schematische Darstellungen des erfindungsgemässen Prinzips mit zwei verschiedenen Stellungen des Versuchstieres,

Fig. 2 ein praktisches Ausführungsbeispiel einer apparativen Realisierung des in den Fig. 1a, 1b gezeigten Prinzips,

Fig. 3 einen Schnitt längs der Linie III-III der Fig. 2,

Fig. 4 ein Blockschaltbild eines ersten Ausführungsbeispiels der elektronischen Auswertungsschaltung,

Fig. 5 ein Blockschaltbild eines zweiten Ausführungsbeispiels der elektronischen Auswertungsschaltung,

Fig. 6 4 Testdiagramme,

Fig. 7 eine schematische Darstellung des erfindungsgemässen Prinzips, bei dem statt eines Schattenbildes ein mit Hilfe einer Optik erzeugtes Projektionsbild erfasst wird, und

Fig. 8 eine Verstärkerschaltung zur Ankopplung der Photoelemente der Vorrichtung an einen angeschlossenen Schreiber, Amplituden-Schwellenwertdetektor oder Analogeingang eines zur Aufzeichnung und Auswertung eingesetzten Computers.

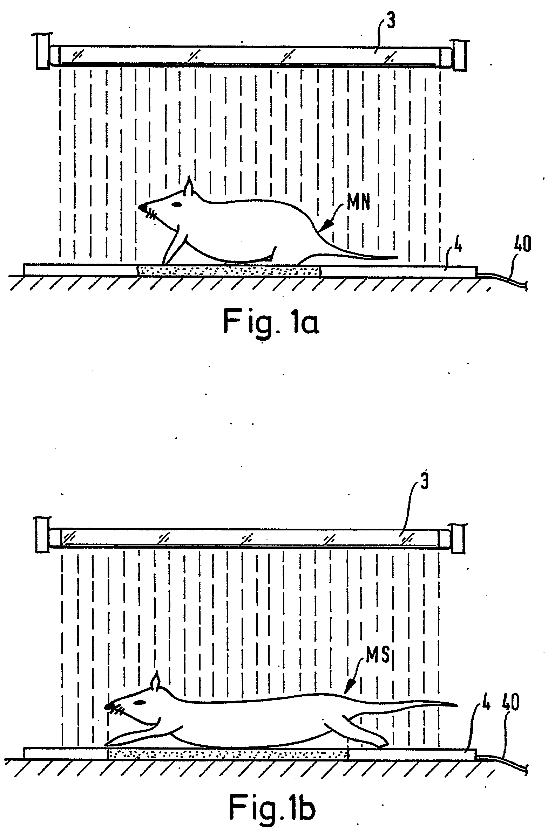

.Die Fig. 1a und 1b zeigen eine von einer Lichtquelle 3 von oben bestrahlte Maus in zwei verschiedenen Stellungen auf einer durch ein Photoelement 4 gebildeten Projektionsfläche, die sich horizontal, d.h. rechtwinklig zur Richtung der Schwerkraft erstreckt, wobei zur Vereinfachung eine das Photoelement 4 schützende und als Unterlage für die Maus dienende transparente und zum Reinigen leicht entfernbare, horizontal angeordnete Platte nicht dargestellt ist. Fig. 1a zeigt die Maus in Normalstellung MN und Fig. 1b in Streckstellung MS. In der Streckstellung MS (Fig. 1b) ist die Schattenfläche der Maus auf der Projektionsfläche des Photoelementes 4 grösser als in der Stellung MN (Fig. 1a). Der von dem Photoelement 4 über ein Kabel 40 abgegebene Strom sinkt daher jedesmal ab, wenn die Maus sich streckt (die Verringerung der Umfänge des Mauskörpers ist vernachlässigbar). Die Auswertung des über das Kabel 40 abgegebenen Signals wird weiter unten ausführlich erläutert. Sie erfolgt durch Aufzeichnen der Bewegungen auf einem Speichermedium und/oder durch Auszählen der relevanten Streckbewegungen mit Hilfe einer Auswertungsschaltung oder eines programmierbaren Computers.

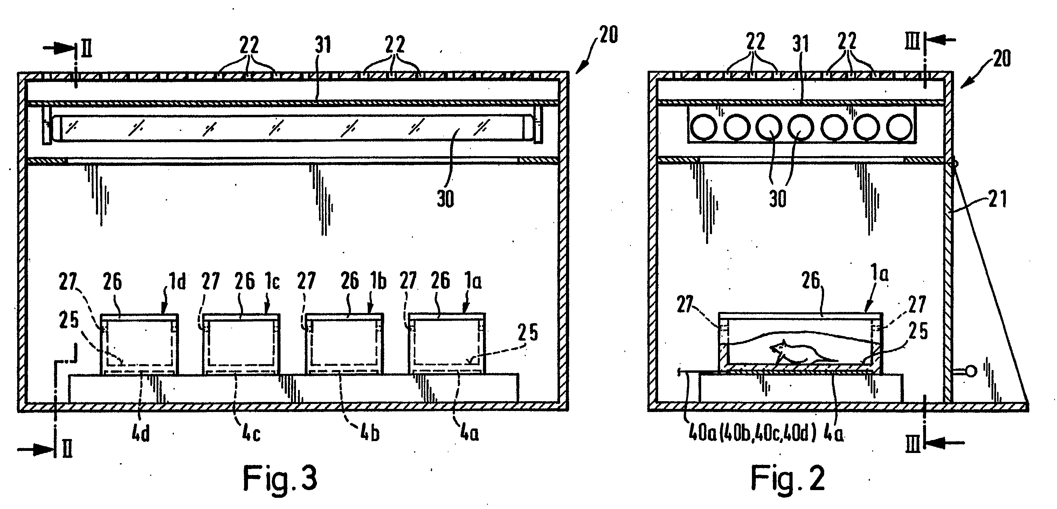

Im praktischen apparativen Ausführungsbeispiel der Fig. 2 und 3 sind in einem oben mit Luftlöchern 22 versehenen Gehäuse 20 vier identisch ausgebildete Einzel-Tierkäfige 1a, 1b, 1c und 1d angeordnet. Jeder dieser Käfige hat einen durchsichtigen Boden 25 unter dem die Photoelement-Projektionsfläche 4a, 4b, 4c bzw. 4d angeordnet ist. Die Käfige 1a bis 1d sind oben mit je einer Glasplatte 26 abgedeckt und seitlich mit Luftlöchern 27 versehen. Das Gehäuse 20 ist bedienungsseitig mit einer Klappe 21 abschliessbar. Als Photoelemente 4a bis 4d sind vorzugsweise Solarzellen eingesetzt.

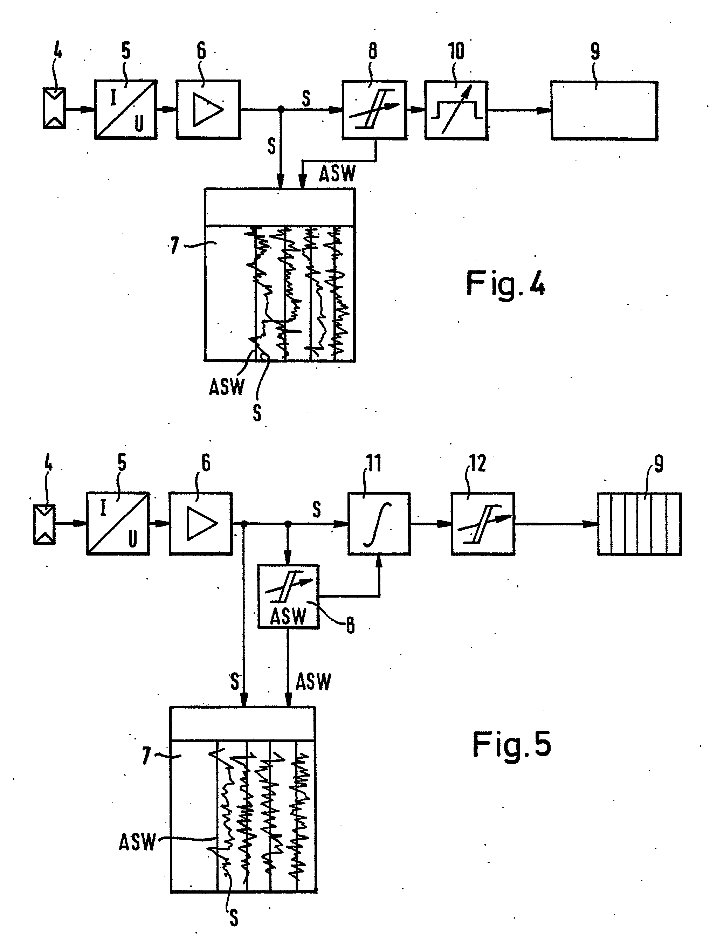

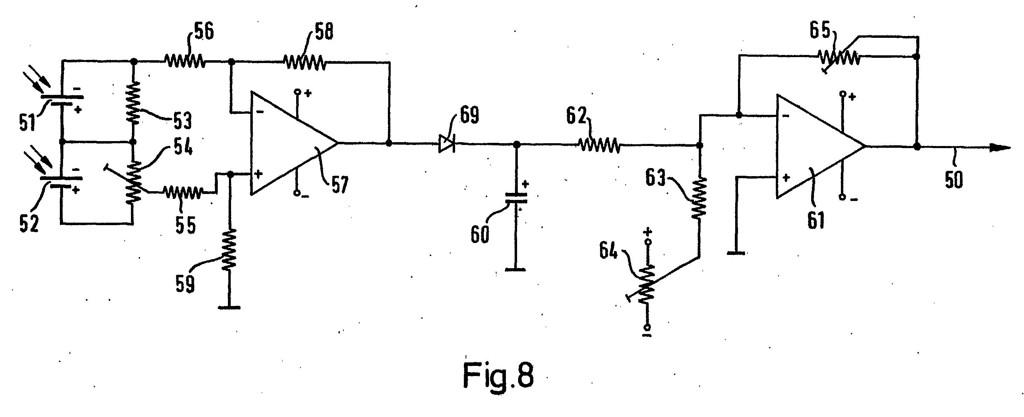

Der von den Photoelementen 4a bis 4d gelieferte Strom gelangt über die Kabel 40a bis 40d zur Auswertungsschaltung gemäss Fig. 4 oder 5, die als erste Stufen eine Verstärkerschaltung gemäss Fig. 8 enthält.

In den Fig. 4 und 5 ist das Photoelement 4 eines jeden der beispielsweise vier vorhandenen Kanäle nochmals symbolisch dargestellt. In beiden Anordnungen wird das von dem Photoelement 4 gelieferte Signal über einen Strom/Spannungsumwandler 5 und einen Verstärker 6 einerseits einem Schreiber 7 und andererseits einem Amplituden-Schwellenwertdetektor 8 mit entsprechend den jeweiligen Versuchsbedingungen einstellbarem Schwellenwert ASW zugeführt. Der jeweils empirisch oder automatisch ermittelte und eingestellte Amplituden-Schwellenwert ASW wird dem Schreiber 7 zugeführt und von diesem gleichzeitig mit dem vom Verstärker 6 eingespeisten Signal S aufgezeichnet.

Gemäss der Schaltungsanordnung der Fig. 4 ist der Ausgang des Amplituden-Schwellenwertdetektors 8 über einen wahlweise einstellbaren Zeitintervall-Schwellenwertdetektor 10 mit einem Zähler 9 mit fix eingestellter Zähldauer angeschlossen. Der Zähler zählt innerhalb der z.B. voreingestellten Zeitdauer von 10 Minuten alle Zustände, in denen das Signal S für eine bestimmte, durch den Zeitintervall-Schwellenwertdetektor 10 festgelegte Mindestdauer von z.B. 1,5 Sekunden einen bestimmten, durch den Amplituden-Schwellenwertdetektor 8 festgelegten unteren Wert unterschreitet (grosser Schatten = kleiner Strom = kleine Spannung = "kleines" Signal S).

Gemäss der Schaltungsanordnung der Fig. 5 ist der Ausgang des Verstärkers 6 mit einem Integrator 11 verbunden, welcher vom Amplituden-Schwellendetektor 8 so gesteuert ist, dass er das vom Verstärker 6 eingespeiste Signal S jeweils über die Zeitintervalle integriert, während denen das Signal S den eingestellten Amplituden-Schwellenelement ASW unterschreitet. Der Ausgang des Integrators 11 ist über einen Integralamplituden-Schwellenwertdetektor 12 mit gleichfalls einstellbarem Schwellenwert mit dem Zähler 9 verbunden. Der Zähler 9 zählt demnach innerhalb einer bestimmten Zeitdauer diejenigen Zustände, in denen die Integralwerte (Ausgangssignale des Integrators 11) den am Integralamplituden-Schwellenwertdetektor 12 voreingestellten Schwellenwert überschreiten.

Die Auswertungsschaltungen der Fig. 4 oder 5 sind in der Praxis mehrkanalig ausgebildet. Für die Käfiganordnung der Fig. 2 und 3 sind (bei Besetzung aller Käfige) vier Kanäle erforderlich.

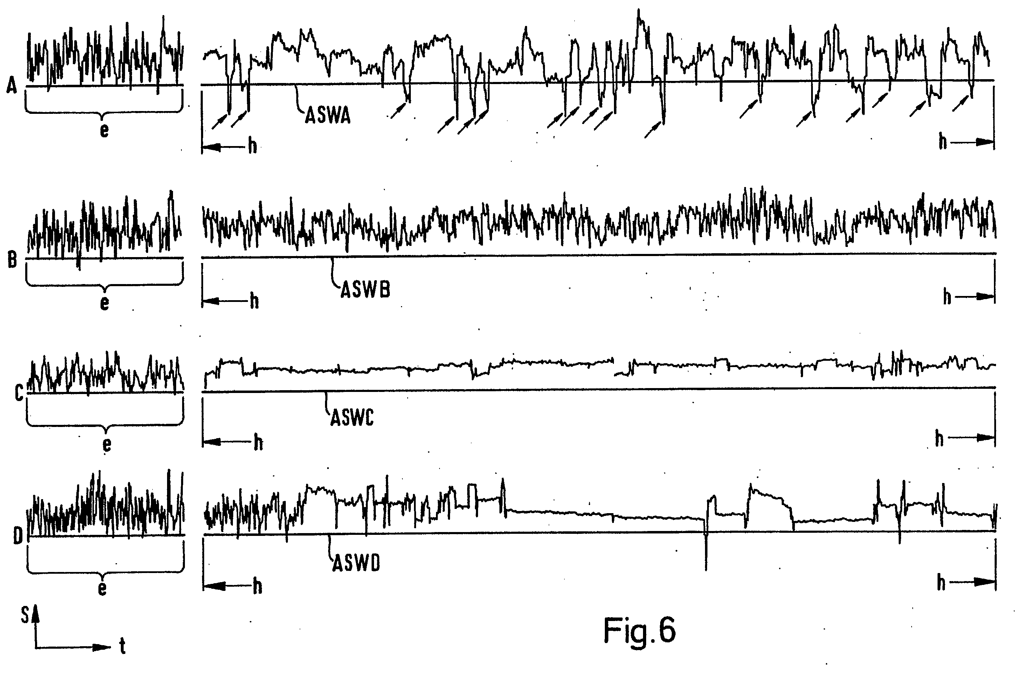

Die Fig. 6 zeigt als Ergebnis eines Testversuches das Kurvenblatt eines an eine Auswertungsschaltung gemäss Fig. 4 oder 5 bzw. an die in Fig. 8 dargestellten Stufen angeschlossenen Vierkanalschreibers.

Die durch die Bewegungen von vier Versuchstieren A bis D erzeugten und verstärkten Signale S sind in den Spuren A bis D aufgezeichnet.

Vor Beginn der Aufzeichnung (des Signals S) werden den Tieren B bis D die verschiedenen, miteinander zu vergleichenden Substanzen verabreicht. Das Tier A (Kontrolltier) bleibt unbehandelt; es erhält lediglich das Lösungsmittel (meist Wasser). Im vorliegenden Versuch wurden den Tieren B bis D die folgenden Substanzen verabreicht:
B Entzündungshemmer (Voltaren®)

C Analgetikum (Morphin)
D anregende Substanz (Amphetamin)

Die Kurvenstücke e zeigen den sogenannten Vorlauf (Dauer ca. 2 Minuten), während dem der Einfluss der etwa 10 Minuten vorher verabreichten Testsubstanzen auf das Vehalten der Tiere aufgezeichnet wird. Der Vorlauf dient zur Festlegung des Amplituden-Schwellenwertes, der dann in der Folge (Hauptlauf) das Kriterium dafür ist, was als Streckbewegung zu werten ist und was nicht. In der Regel wird der Amplituden-Schwellenwert ASW als Mittelwert z.B. der 3.- bis 10.-höchsten Ausschläge (nach unten) festgelegt. Dieser Schwellenwert ASWA bis ASWD (für jedes Tier individuell oder für alle Tiere gleich) wird sodann am Schwellenwertdetektor 8 für jeden Kanal manuell eingestellt oder aber bei Einsatz eines Computers durch ein Auswerteprogramm ermittelt und bei der Verarbeitung des Signals S im Computer verwendet.

Ein Vergleich der Kurvenabschnitte e der Kurven B, C und D mit Abschnitt e der Kurve A zeigt: B Voltaren® hat keinen Einfluss auf die Motilität (Beweglichkeit); C geringe sedierende Wirkung von Morphin; D Frequenzerhöhung, zentral-nervöse Nebenwirkung durch Amphetamin.

Am Ende des Vorlaufs e wird allen Tieren A-D der Reizstoff Phenyl-p-Benzochinon in das Peritoneum injiziert. (Hendershot and Forsaith, Journal Pharmacol. exp. Therap. 125, 237, 1959). Die Wirkung auf die einzelnen Tiere zeigen die Abschnitte h (Hauptversuch) der Kurven A-D (Zeitdauer je ca. 10 Minuten).

Die Kurve A (Kontrolltier ohne Hemmsubstanz) unterschreitet im Abschnitt h den Schwellenwert ASWA insgesamt vierundzwanzigmal, wogegen die Versuchstiere B und C den Schwellenwert ASWB bzw. ASWC im Abschnitt h nicht und das Versuchstier D den Schwellenwert ASWD viermal unterschreitet. Als signifikant bewertet werden jedoch nur solche Überschreitungen, welche der Zeitintervall-Schwellenwertdetektor 10 zum Zähler 9 durchlässt. Wird dieser Schwellenwert z.B. auf 1,5 Sekunden (empirisch ermittelter Optimalwert) eingestellt, dann werden für das Kontrolltier A siebzehn signifikante Unterschreitungen bzw. signifikante Streckungen gezählt und für die Versuchstiere B und C Null Unterschreitungen sowie für das Versuchstier D zwei Unterschreitungen. In der Fig. 6 sind die signifikanten Unterschreitungen durch Pfeile markiert.

Aus den Kurven kann "abgelesen" werden, dass das peripher wirkende Voltaren® (Tier/Kurve B) vollständig vor Schmerzen schützt (Kurve B: Null signifikante Streckbewegungen in Kurvenabschnitt h), ohne die Motilität zu beeinflussen (Kurve B: Kurvenabschnitte e und h bezüglich Amplituden und Frequenzen nahezu identisch). Hingegen beeinflussen zentral wirksame Substanzen die Motilität. Zentral wirksame Analgetika haben eine geringe sedierende Wirkung und verringe rn die Motilität (Tier/Kurve C: Morphin, Amplituden und Frequenzen kleiner). Anregende Substanzen verursachen zentral-nervöse Nebenwirkungen mit veränderter Mortilität (Tier/Kurve D: Amphetamin, Frequenzen in Kurvenabschnitt e erhöht).

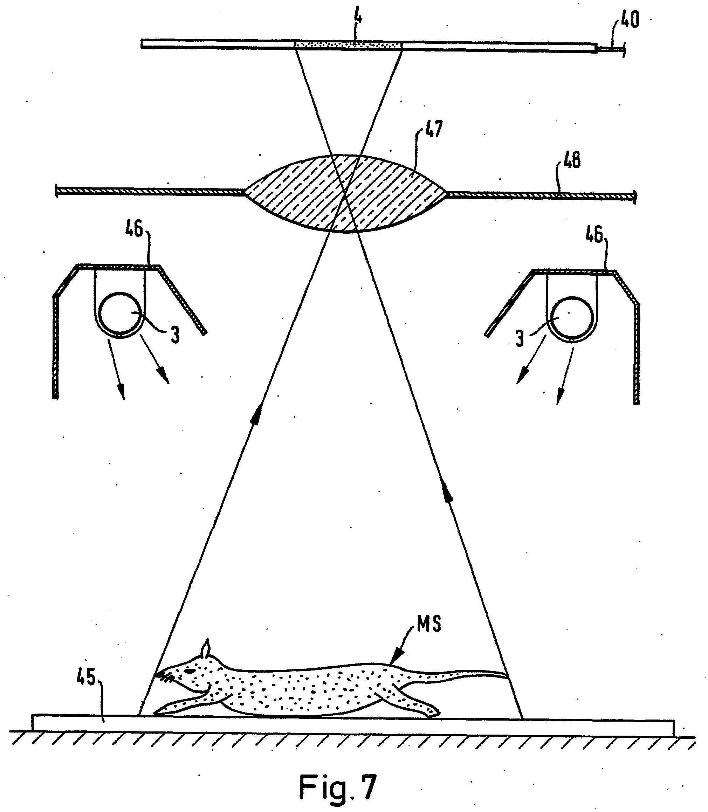

Bei dem in Fig. 7 dargestellten Ausführungsbeispiel der Erfindung ist abweichend von der Anordnung in den Figuren 1a und 1b das Photoelement 4, bei dem es sich wiederum um eine grossflächige Solarzelle handeln kann, oberhalb der im gestreckten Zustand dargestellten Maus MS angeordnet. In Fig. 7 erkennt man zwei Leuchtstofflampen 3 zur Beleuchtung der Maus MS und einer Bodenplatte 45, die sich in ihrer Farbe von der Farbe der Maus MS möglichst stark unterscheidet, um zu erreichen, dass eine möglichst kontrastreiche Szene entsteht. Abschirmungen 46 um die Leuchtstofflampen 3 verhindern einen direkten Lichteinfall des von den Leuchtstofflampen 3 ausgehenden Lichtes auf das Photoelement 4, auf dem mit Hilfe einer Optik 47 ein scharfes Abbild der Maus MS erzeugt wird. Die Optik 47 ist in Fig. 7 schematisch zusammen mit einer Abschirmblende 48 dargestellt. Entsprechend dem Photoelement 4 in den Fig. 1a und 1b ist das Photoelement 4 in Fig. 7 über ein Kabel 40 mit dem Eingang der Auswertungsschaltung gemäss den Figuren 4 oder 5 bzw. über eine Verstärkerschaltung gemäss Fig. 8 mit einem Computer verbunden. Das auf dem Photoelement 4 erzeugte Abbild der Maus MS entspricht dem Schattenbild, das mit einer Anordnung gemäss den Fig. 1a und 1b erzeugt werden kann, wobei jedoch zur Erzielung eines hohen Signal/Rausch-Verhältnisses ein guter farblicher Kontrast zwischen der Maus MS und der Bodenplatte 45 erforderlich ist.

In Fig. 8 ist eine Verstärkerschaltung dargestellt, die dem Strom/Spannungswandler 5 und dem Verstärker 6 in den Fig. 4 und 5 entspricht. Während in den Fig. 4 und 5 der Ausgang 50 der Verstärkerschaltung neben dem Schreiber 7 eine festverdrahtete Auswertungsschaltung mit hardwaremässig realisierten Komponenten speist, ist die Verstärkerschaltung gemäss Fig. 8 gemäss einem weiteren Ausführungsbeispiel der Erfindung unmittelbar mit dem Eingang eines Analogkanals eines Computers verbunden, der über weitere Eingangskanäle verfügt, an denen entsprechende Verstärkerschaltungen angeschlossen sind.

Das am Ausgang 50 anliegende Signal S wird im Computer digitalisiert und unter der Kontrolle des Auswertungsprogramms in einer Weise ausgewertet, die der Funktion der Schaltungen gemäss den Fig. 4 und 5 entspricht.

Der Algorithmus des Programms zur Detektierung von relevanten Streckungen sieht vor, dass das Schattensignal während zwei Minuten in einem Vorlauf aufgenommen und danach während 10 Minuten für den Versuchslauf erfasst und ausgewertet wird. Die Abtastung des Signals erfolgt mit zehn Werten pro Sekunde mit einer Auflösung von 12 Bit. Die Messwerte werden während der Messung im Programm gespeichert und am Ende des Versuchs ausgewertet. Der Computer verfügt über eine Diskettenstation, die es gestattet, wahlweise auf der Diskette nur die Resultate der Auswertung zu speichern oder zusätzlich das Messignal selbst.

Aus den Daten des Vorlaufs wird mit Hilfe des Computerprogramms ein Referenzpegel, der dem Schwellenwert des Amplituden-Schwellendetektors 8 entspricht, für die Detektierung der Streckungen während des sich an den Vorlauf anschliessenden Versuchslaufes bestimmt. Während des Vorlaufs wird dazu der Signalverlauf des am Ausgang 50 vorliegenden Signals auf Maximalwerte untersucht. Ein Maximalwert wird dann akzeptiert, wenn die Differenz zum vorangehenden Minimum eine Minimalamplitude aufweist. Der Schwellenwert wird als der Mittelwert des 6.grössten bis 10.grössten Maximums festgelegt.

Der Algorithmus zur Detektierung und Bestimmung der Streckungen sieht vor, dass das Signal am Ausgang 50 während einer Mindestzeit den Schwellenwertes überschreitet. Die vom Signal und vom Schwellenwert eingeschlossene Fläche muss grösser als die Fläche eines Rechtecks derselben Länge und einer vorgegebenen Minimalhöhe sein. Die "Aktivität" des Versuchstieres wird durch die "Frequenz" und "Amplitude" des Signals bestimmt. Als charakteristisches Merkmal dafür dient die "Kurvenlänge". Bei der Signalauswertung werden Paare von Minima/Maxima gesucht und deren Differenz wird für die ganze Messung aufsummiert. Diese durch die Messzeit dividierte Summe wird schliesslich bei der Auswertung als Aktivität ausgegeben.

Wie man in Fig. 8 erkennt, ist der Ausgang des Operationsverstärkers 57 über eine Diode 69 mit einem Elektrolytkondensator 60 verbunden. Die Dimensionierung der Bauteile ist so gewählt, dass bei Beaufschlagen der Solarzelle mit Licht bei Abwesenheit des durch die Versuchstiere erzeugten Schattens eine Spannung von 4,2 Volt am Elektrolytkondensator auftritt. Die durch den Elektrolytkondensator 60 bestimmte Zeitkonstante ist vorzugsweise kleiner als eine Sekunde aber so gross, dass die Intensitätsschwankungen der Leuchtstofflampen 3 sich nicht mehr störend als ein überlagertes Wechselstromsignal bemerkbar machen. Wenn die Fläche der Solarzelle mehr oder weniger stark durch ein Versuchstier abgeschattet werden, sinkt die Spannung am Elektrolytkondensator 60, wobei eine Signalglättung auftritt.

Die am Elektrolytkondensator 60 anliegende Spannung gelangt über einen Koppelwiderstand 62 zu einem Impedanzwandler, der einen dem Operationsverstärker 57 entsprechenden Operationsverstärker 61 enthält. Der mit dem Koppelwiderstand 62 verbundene invertierende Eingang des Operationsverstärkers 61 ist auch über einen Widerstand 63 mit dem Abgriff eines Potentiometers 64 verbunden, an den eine aus den Betriebsspannungen abgeleitete Ueberlagerungsspannung abgreifbar ist, um eine Nullpunkteinstellung vornehmen zu können. Der invertierende Eingang des Operationsverstärkers 61 ist ausserdem über ein Potentiometer 65 mit dem Ausgang des Operationsverstärkers 61 verbunden. Das Potentiometer 65 dient zur Einstellung der Verstärkung. Der nichtinvertierende Eingang des Operationsverstärkers 61 liegt an Masse, wie der Fig. 8 entnommen werden kann.

Die in Fig. 8 gezeigte Verstärkerschaltung bildet somit zusammen mit einer Solarzelle (51) eine äußerst einfach aufgebaute Schaltung zur Erfassung der Motilität eines Versuchstieres, wobei es möglich ist, eine Vielzahl von verschieden grossen Schattenflächen zu erfassen, ohne dass Signalsprünge infolge einer Vielzahl von diskreten einzelnen Bildpunkten zugeordneten Photodetektoren auftreten, die einen hohen Aufwand bei der Signalverarbeitung erfordern und infolge der Diskretisierung in eine Vielzahl von Bildpunkten eine Verschlechterung des Auflösungsvermögens mit sich bringen.

**Patentansprüche**

1. Verfahren zur Aufzeichnung und/oder Auswertung des Streck- und Bewegungsverhaltens von Versuchstieren, insbesondere Mäusen, wobei mittels einer Strahlung, welche der Tierkörper zumindest teilweise absorbiert und/oder reflektiert, ein Bild des Tierkörpers auf einer Projektionsfläche erzeugt und photoelektrisch ein Signal generiert sowie aufgezeichnet und weiterverarbeitet wird, welches proportional zu der vom Bild des Tierkörpers auf der Projektionsfläche eingenommenen Fläche ist, dadurch gekennzeichnet, dass als Projektionsfläche ein grossflächiges, die Bildfläche homogen abdeckendes Photoelement, insbesondere eine Solarzelle, verwendet wird.

2. Verfahren nach Anspruch 1, dadurch gekennzeichnet, dass die Strahlung von oben erfolgt und die Projektionsfläche unterhalb des auf einer horizontalen Ebene abgestützten Tieres angeordnet wird, so dass der Tierkörper als Schattenbild und im wesentlichen im "Grundriss" abgebildet, wird.

3. Verfahren nach Anspruch 1 oder 2, dadurch gekennzeichnet, dass für das Signal ein Schwellenwert der Amplitude festgelegt und die Anzahl der Unterschreitungen dieses Schwellenwertes innerhalb einer bestimmten Zähldauer gezählt werden.

4. Verfahren nach Anspruch 1 oder 2, dadurch gekennzeichnet, dass für das Signal ein Schwellenwert der Amplitude und für die Zeitintervalle, in welchen das Signal diesen Amplituden-Schwellenwert unterschreitet, ein Zeitintervall-Schwellenwert festgelegt wird und dass innerhalb einer bestimmten Zähldauer diejenigen Zustände gezählt werden, in denen der Amplituden-Schwellenwert unterschritten und gleichzeitig der Zeitintervall-Schwellenwert überschritten wird.

5. Verfahren nach Anspruch 1 oder 2, dadurch gekennzeichnet, dass das Signal jeweils über die Zeitintervalle, während denen es einen bestimmten Amplituden-Schwellenwert unterschreitet, integriert wird; dass für diese Integrale ein Integral-Schwellenwert festgelegt wird; und dass innerhalb einer bestimmten Zeitdauer diejenigen Zustände gezählt werden, in denen die Integrale den Integral-Schwellenwert übersteigen.

6. Verfahren nach Anspruch 4 oder 5, dadurch gekennzeichnet, dass die Schwellenwerte gemäss den jeweiligen Versuchsbedingungen eingestellt und ermittelt werden, indem während eines Vorlaufs der Mittelwert einer bestimmten Anzahl von z.B. zehn grössten Signalausschlägen unter Ausschluss einer bestimmten kleineren Anzahl von z.B. drei bis sechs der allergrössten dieser grössten Signalausschläge gebildet wird.

7. Verfahren nach Anspruch 4, dadurch gekennzeichnet, dass der Zeitintervall-Schwellenwert auf etwa 0,5 sec bis 4 sec, vorzugsweise 1,5 sec eingestellt wird.

8. Vorrichtung zur Durchführung des Verfahrens nach Anspruch 1, mit einem Tierkäfig (1), dessen Boden (25) lichtdurchlässig und horizontal verlaufend gestaltet ist, und in den von oben das Licht einer Lichtquelle eintritt, und mit einer photoelektrischen Detektoranordnung, welche über einen Verstärker (6) an ein Signalaufzeichnungsgerät (7) und/oder eine Auswerteeinrichtung (8, 9, 10, 11, 12) angeschlossen ist, dadurch gekennzeichnet, dass die photoelektrische Detektoranordnung ein einzelnes Photoelement (4) umfasst, welches einen Projektionsschirm bildet, der unter dem Boden (25) angeordnet ist oder den Käfigboden (25) bildet.

9. Vorrichtung zur Durchführung des Verfahrens nach Anspruch 1 mit einem im Innern hellen Tierkäfig, dessen Bodenbereich (45) mit Hilfe einer Optik (47) auf einer im Abstand vom Boden des Käfigs angeordneten lichtempfindlichen Detektoranordnung abbildbar ist, durch die ein Signal in Abhängigkeit von den Bewegungen eines in dem Käfig eingeschlossenen Versuchstieres zur Registrierung und Auswertung der Bewegungsaktivität des Versuchstieres erzeugbar ist, dadurch gekennzeichnet, dass die Detektoranordnung ein die Bildfläche der Optik homogen abdeckendes Photoelement (4) aufweist.

10. Vorrichtung nach Anspruch 8 oder 9, dadurch gekennzeichnet, dass das Photoelement (4) durch eine einzige grossflächige Solarzelle (51) gebildet ist.

11. Vorrichtung nach Anspruch 8 oder 9, dadurch gekennzeichnet, dass das Photoelement (4) über den Verstärker (6) und einen Amplituden-Schwellenwertdetektor (8) mit einstellbarem Schwellenwert (ASW) an einen Zähler (9) angeschlossen ist.

12. Vorrichtung nach Anspruch 11, dadurch gekennzeichnet, dass das Signalaufzeichnungsgerät ein Schreiber (7) ist, der so mit dem Amplituden-Schwellenwertdetektor (8) verbunden ist, dass er den jeweils eingestellten Amplituden-Schwellenwert (ASW) mitschreibt.

13. Vorrichtung nach Anspruch 11, dadurch gekennzeichnet, dass der Amplituden-Schwellenwertdetektor (10) an den Zähler (9) angeschlossen ist.

14. Vorrichtung nach Anspruch 11, dadurch gekennzeichnet, dass der Amplituden-Schwellenwertdetektor (8) über einen Integrator (11) und einem diesem nachgeschalteten Integral-Schwellenwertdetektor (12) an den Zähler (9) angeschlossen ist.

15. Vorrichtung nach einem der Ansprüche 8 bis 14, dadurch gekennzeichnet, dass mehrere Käfige (1) die jeder ein einzelnes Photoelement (4) umfassen, in einem Gehäuse (20) angeordnet sind, in dessen Oberteil eine gemeinsame Lichtquelle, vorzugsweise Leuchtstoffröhren (30) mit Reflektoren (31) für alle Käfige (1) angeordnet ist.

16. Vorrichtung nach Anspruch 15, dadurch gekennzeichnet, dass der Schreiber (7) ein Mehrfachschreiber ist und dass jedes Photoelement (4) auf einen der Eingänge dieses Mehrfachschreibers geschaltet ist.

17. Vorrichtung nach Anspruch 8 oder 9, dadurch gekennzeichnet, dass das Signalaufzeichnungsgerät und die Auswerteeinrichtung durch einen Computer gebildet sind, durch den die über eine Verstärkerschaltung eingespeisten Signale analysierbar sind.

## Claims

1. Process for the recording and/or evaluation of the stretching and moving behaviour of experimental animals, in particular mice, wherein by means of a radiation which is absorbed and/or reflected at least in part by the body of the animal, an image of the animal body is produced on a projection surface and a signal is generated photoelectrically and also recorded and processed further, which signal is proportional to the area taken up on the projection surface by the image of the body of the animal, characterised in that a large-area photoelement homogenously covering the image area, in particular a solar cell, is used as the projection surface.

2. Process according to Claim 1, characterised in that the radiation is applied from above and the projection surface is located under the animal, which is supported on a horizontal plane, so that the body of the animal is reproduced as a shadow image and essentially in "projection".

3. Process according to Claim 1 or 2, characterised in that a threshold value of the amplitude is determined for the signal and the number of times that the value drops below this threshold value within a given counting period is counted.

4. Process according to Claim 1 or 2, characterised in that a threshold value of the amplitude is determined for the signal and a time interval threshold value is determined for the time intervals in which the signal drops below this amplitude threshold value, and that within a given counting period the states are counted in which the value falls below the amplitude threshold value and at the same time the time interval threshold value is exceeded.

5. Process according to Claim 1 or 2, characterised in that the signal is integrated each time over the time intervals during which it falls below a given amplitude threshold value; that an integral threshold value is determined for these integrals; and that within a certain period of time the states wherein the integrals exceed the integral threshold value are counted.

6. Process according to Claim 4 or 5, characterised in that the threshold values are set and determined in keeping with the prevailing experimental conditions, in that during a pre-run the mean value of a certain number of, for instance, ten of the largest signal deflections is formed, with the exclusion of a certain smaller number, for instance three to six, of the very largest of said largest signal deflections.

7. Process according to Claim 4, characterised in that the time interval threshold value is set to approximately 0.5 sec to 4 sec, preferably 1.5 sec.

8. Apparatus for carrying out the process according to Claim 1, with an animal cage (1), the base (25) of which is light-permeable and extends horizontally, and into which the light from a light source enters from above, and with a photoelectric detector layout which is connected to a signal recording device (7) and/or an evaluating device (8, 9, 10, 11, 12) via an amplifier (6), characterised in that the photoelectric detector layout comprises a single photoelement (4) which forms a projection screen which is arranged under the base (25) or constitutes the base (25) of the cage.

9. Apparatus for carrying out the process according to Claim 1, with an internally illuminated animal cage, the base area (45) of which can be reproduced on a photosensitive detector layout located at a distance from the base of the cage with the aid of an optical device (47), whereby a signal may be produced as a function of the movements of an experimental animal shut in the cage, to record and evaluate the movement activity of said experimental animal, characterised in that the detector layout comprises a photoelement (4) homogenously covering the image area of the optical device.

10. Apparatus according to Claim 8 or 9, characterised in that the photoelement (4) is formed by a single, large-area solar cell (51).

11. Apparatus according to Claim 8 or 9, characterised in that the photoelement (4) is connected to a counter (9) via the amplifier (6) and an amplitude threshold value detector (8) with an adjustable threshold value (ASW).

12. Apparatus according to Claim 11, characterised in that the signal recording device is a recorder (7) connected to the amplitude threshold value detector (8) in a manner such that it simultaneously also writes the amplitude threshold value (ASW) set in each case.

13. Apparatus according to Claim 11, characterised in that the amplitude threshold value detector (8) is connected to the counter (9) via a time interval threshold value detector (10).

14. Apparatus according to Claim 11, characterised in that the amplitude threshold value detector (8) is connected io the counter (9) via an integrator (11) and a subsequent integral threshold value detector (12).

15. Apparatus according to one of Claims 8 to 14, characterised in that several cages (1), which each comprise a single photoelement (4), are located in a housing (20), in the upper part of which a common light source, preferably fluorescent tubes (30) with reflectors (31), is arranged for all of the cages (1).

16. Apparatus according to Claim 15, characterised in that the recorder (7) is a multiple recorder and that each photoelement (4) is connected to one of the inlets of said multiple recorder.

17. Apparatus according to Claim 8 or 9, characterised in that the signal recording device and the evaluating device are formed by a computer through which the signals fed in through an amplifier circuit may be analysed.

## Revendications

1. Procédé d'enregistrement et/ou d'évaluation du comportement d'extension et de mobilité d'animaux de laboratoire, en particulier de souris, dans lequel on forme une image du corps de l'animal sur une surface de projection au moyen d'un rayonnement que le corps de l'animal absorbe et/ou réfléchit au moins partiellement et un signal photoélectrique est généré, enregistré et traité, lequel signal est proportionnel à la surface prise par l'image du corps de l'animal sur la surface de projection, caractérisé en ce qu'on utilise comme surface de projection un élément photoélectrique à grande surface, couvrant de manière homogène le champ d'image, en particulier une cellule solaire.

2. Procédé selon la revendication 1, caractérisé en ce que le rayonnement est émis par le haut et la surface de projection est située au-dessous de l'animal reposant sur un plan horizontal de sorte que l'image du corps de l'animal soit représentée en tant que silhouette et essentiellement sur un plan horizontal.

3. Procédé selon la revendication 1 ou 2, caractérisé en ce qu'une valeur de seuil de l'amplitude est déterminée pour le signal que l'on compte et le nombre de fois où le signal est inférieur à cette valeur de seuil pendant une période de comptage déterminée.

4. Procédé selon la revendication 1 ou 2, caractérisé en ce qu'une valeur de seuil de l'amplitude est déterminée pour le signal et une valeur de seuil d'intervalle de temps est définie pour l'intervalle de temps pendant lequel le signal est inférieur à ladite valeur de seuil d'amplitude, et en ce que l'on compte pendant une durée de comptage déterminée les états dans lesquels la valeur de seuil d'amplitude n'est pas atteinte tandis que, simultanément, la valeur de seuil d'intervalle de temps est dépassée.

5. Procédé selon la revendication 1 ou 2, caractérisé en ce que le signal est intégré pendant les intervalles de temps durant lesquels il reste inférieur à une certaine valeur de seuil d'amplitude, qu'une valeur de seuil intégrée est déterminée pour ces intégrales, et qu'on compte pendant une durée déterminée les états dans lesquels les intégrales dépassent la valeur de seuil intégrée.

6. Procédé selon la revendication 4 ou 5, caractérisé en ce que les valeurs de seuil sont ajustées et déterminées en fonction des conditions opératoires d'essai respectives par formation, au cours d'une phase préalable, de la valeur moyenne d'un certain nombre d'amplitudes de signaux, par exemple des dix plus fortes amplitudes de signaux, en excluant un nombre déterminé, plus faible, par exemple trois à six amplitudes de signaux, des plus fortes de ces dix amplitudes maximales.

7. Procédé selon la revendication 4, caractérisé en ce que la valeur de seuil d'intervalle de temps est ajustée à une valeur comprise approximativement entre 0,5 et 4 secondes, de préférence 1,5 seconde.

8. Dispositif pour la mise en œuvre du procédé selon la revendication 1, comportant une cage d'animal (1) dont le fond (25) est transparent et s'étend à l'horizontale, dans laquelle pénètre, par le haut, la lumière d'une source lumineuse, et un dispositif détecteur photoélectrique qui est relié au moyen d'un amplificateur (6) à un appareil d'enregistrement de signaux (7) et/ou à un dispositif d'évaluation (8, 9, 10, 11, 12), caractérisé en ce que le dispositif détecteur photoélectrique comprend un seul élément photoélectrique (4) qui forme un écran de projection qui est disposé sous le fond (25) ou forme le fond de cage (25).

9. Dispositif pour la mise en œuvre du procédé selon la revendication 1, comportant une cage d'animal dont l'intérieur est clair, une image de la zone de fond (45) pouvant être formée au moyen d'un dispositif optique (47) sur un dispositif détecteur sensible à la lumière, situé à distance du fond de la cage et au moyen duquel peut être produit un signal en fonction des déplacements d'un animal de laboratoire enfermé dans la cage, pour enregistrer et analyser la mobilité dudit animal de laboratoire, caractérisé en ce que le dispositif détecteur comporte un élément photoélectrique (4) qui couvre de manière homogène le champ d'image du dispositif optique.

10. Dispositif selon la revendication 8 ou 9, caractérisé en ce que l'élément photoélectrique (4) est formé d'une seule cellule solaire (51) à grande surface.

11. Dispositif selon la revendication 8 ou 9, caractérisé en ce que l'élément photoélectrique (4) est relié à un compteur (9) au moyen de l'amplificateur (6) et d'un détecteur de valeur de seuil d'amplitude (8) ayant une valeur de seuil d'amplitude (VSA) réglable.

12. Dispositif selon la revendication 11, caractérisé en ce que l'appareil d'enregistrement de signaux est un enregistreur (7) qui est relié au détecteur de valeur de seuil d'amplitude (8) de manière à ce qu'il puisse enregistrer la valeur de seuil d'amplitude (VSA) ajustée.

13. Dispositif selon la revendication 11, caractérisé en ce que le détecteur de valeur de seuil d'amplitude (8) est relié au compteur (9) au moyen d'un détecteur de valeur de seuil d'intervalle de temps (10).

14. Dispositif selon la revendication 11, caractérisé en ce que le détecteur de valeur de seuil d'amplitude (8) est relié au compteur (9) au moyen d'un intégrateur (11) et d'un détecteur de valeur de seuil intégrée (12) qui est connecté en aval dudit intégrateur (11).

15. Dispositif selon l'une des revendications 8 à 14, caractérisé en ce que plusieurs cages (1), qui comportent chacune un seul élément photoélectrique (4), sont disposées dans une enceinte (20) dans la partie supérieure de laquelle est disposée une source lumineuse commune à toutes les cages (1), de préférence des tubes fluorescents (30) avec des réflecteurs (31).

16. Dispositif selon la revendication 15, caractérisé en ce que l'enregistreur (7) est un enregistreur multicourbe et chaque élément photoélectrique (4) est connecté à une des entrées dudit enregistreur multicourbe (7).

17. Dispositif selon la revendication 8 ou 9, caractérisé en ce que l'appareil d'enregistrement de signaux et le dispositif d'évaluation sont constitués par un ordinateur au moyen duquel les signaux amenés par un montage amplificateur peuvent être analysés.

Fig. 1a

Fig.1b

Fig.3

Fig.2

EP 0 263 062 B1

Fig. 4

Fig. 5

Fig.6

Fig. 7

Fig.8

EP 0 263 062 B1